# EUROPEAN PATENT SPECIFICATION

(11) **EP 2 201 914 B1**
(45) Date of publication and mention of the grant of the patent: **05.12.2018**
(21) Application number: 08840775.4
(22) Date of filing: 15.10.2008
(51) Int. Cl.: A61F 2/915, A61F 2/89, A61L 31/06, A61L 31/14

(54) **TUBE-FORMING ELEMENT FOR VASCULAR STENT AND VASCULAR STENT**
ROHRFORMENDES ELEMENT FÜR EINEN GEFÄSSSTENT UND GEFÄSSSTENT
ÉLÉMENT DE FORMATION DE TUBE POUR UN STENT VASCULAIRE ET STENT VASCULAIRE

(30) Priority: 16.10.2007 JP 2007269339
(43) Date of publication of application: 30.06.2010
(73) Proprietor: Kabushiki Kaisha Kyoto Iryo Sekkei, Yamashina-ku Kyoto-shi Kyoto 607-8035 (JP)
(72) Inventor: IGAKI, Keiji, Kyoto-shi Kyoto 607-8035 (JP)
(74) Representative: Müller Hoffmann & Partner
(86) International application number: PCT/JP2008/002923
(87) International publication number: WO 2009/050888

(56) References cited:
- WO-A1-99/43272
- WO-A1-2006/051912
- GB-A- 2 347 861
- JP-A- 10 286 312
- JP-A- 2003 052 834
- JP-A- 2007 244 749
- US-A- 5 817 152

## Description

### TECHNICAL FIELD

This invention relates to a cylindrically-shaped element for a luminal stent deployed in a vessel in a living vessel, such as a blood vessel, trachea or biliary duct to scaffold the inner lumen of the vessel from inside, and relates to a luminal stent formed by using this cylindrically-shaped element.

### BACKGROUND ART

Heretofore, when stenosis occurs in a vessel of a living body such as a blood vessel including an artery, percutaneous transluminal angioplasty (PTA) is performed in which the stenosed portion in the vessel is expanded to improve the blood flow by inserting a balloon provided in the vicinity of the end of a balloon catheter and then inflating the balloon which is contracted initially.

It is known that despite PTA applied initially, stenosis tends to recur at a high probability in the once stenosed site. The current practice for prevention of such restenosis is to implant a cylindrical stent in the site treated with PTA. The stent is intended to scaffold a blood vessel from inside for the purpose of prevention of restenosis therein by being inserted into the blood vessel with its contracted state and subsequently expanded to be implanted therein.

As such a sort of stent, a stent comprised of a cylindrical metal member with slits to permit its expansion or contraction in diameter has been currently known.

Meanwhile, use of metal stents may lead to foreign-body reaction due to its long term presence in a living body, it is therefore not appropriate to leave them therein semi-permanently. Moreover, metal stents in need of removal after its deployment in a living body surgical procedures that impose severe burden on the patient.

To solve such inherent problems with metal stents, the present inventor has proposed a stent made of a biodegradable polymer in International Patent Publication Nos. WO92/15342 (Patent Document 1) and WO00/13737 (Patent Document 2).

A stent made of a biodegradable polymer has also been proposed in JP Laid-Open Patent Publication Nos. H-11-57018 (Patent Document 3) and 2004-33579 (Patent Document 4).
Patent Document 1: International Patent Publication No. WO92/15342
Patent Document 2: International Patent Publication No. WO00/13737
Patent Document 3: JP Laid-Open Patent Publication No. H-1 1-57018
Patent Document 4: JP Laid-Open Patent Publication No. 2004-33579.

GB2347861 discloses a cylindrical-shaped element and a luminal stent formed with such cylindrical-shaped elements.

### DISCLOSURE OF THE INVENTION

### PROBLEMS TO BE SOLVED BY THE INVENTION

Meanwhile, it is desirable that a stent implanted in a vessel of a living body have such flexibility that it deforms easily in accordance with a sinuous or meandering vessel in order to achieve smooth insertion into a vessel.

A region in need of scaffold by a stent for the purpose of prevention of restenosis varies depending on condition of a vessel in which the stent is to be implanted. Therefore, to achieve a suitable expansion and scaffold in accordance with a status of a vessel in which the stent is to be implanted, the stent is required to be provided in multiple lengths and various diameters.

It is an object of the present invention to provide a cylindrically-shaped element capable of constituting a luminal stent having excellent flexibility to facilitate smooth insertion into a vessel, and a luminal stent using this cylindrically-shaped element.

It is another object of the present invention to provide a cylindrically-shaped element which facilitates production of luminal stents provided in various lengths and various diameters and a luminal stent using this cylindrically-shaped element.

It is still another object of the present invention to provide a cylindrically-shaped element for a luminal stent advantageously using a biodegradable polymer to reduce burden which a stent may impose on the patient, and a luminal stent using this cylindrically-shaped element.

### MEANS FOR SOLVING THE PROBLEMS

A cylindrically-shaped element for a luminal stent according to the present invention constitutes a luminal stent of cylindrical structure, and is made of a strand of a biodegradable polymer, wherein the strand is formed into a cylindrical shape by being repeatedly bent such that a linear part and a bend part alternate in sequence and non-interruptedly connecting one end of said strand to the other end, as disclosed in claim 1.

In one embodiment of the strand constitute the cylindrically-shaped element which is formed into a cylindrical shape, one end of the strand is non-interruptedly connected to the other end by being held with a cylindrical connecting member of a biodegradable polymer.

The connecting member used herein is desirably formed of the same type of biodegradable polymer used in the strand and in addition, desirably caulked thermally to hold both ends of the strand.

The connecting member for connecting one end of the strand to the other may connect both ends of the strand by being welded at least partially.

Additionally the strand is formed so as to have tapered ends that are overlapped with each other to be non-interruptedly connected together; therefore the strand may avoid to be thicker at the overlapped section than the other sections of the strand.

To make the strand, a non-interrupted continuous monofilament or a multifilament made up of a plurality of monofilaments unified together may be used. These monofilament and multifilament may be made by melt-spinning a biodegradable polymer.

The strand is made of one or more biodegradable polymers selected from a group of polylactic acid (PLLA), polyglycolic acid (PGA), a copolymer of polyglycolic acid and polylactic acid, polydioxanone, a copolymer of trimethylene carbonate and glycolide, and a copolymer of polyglycolic acid or polylactic acid and ε -caprolactone.

The present invention also provides a luminal stent of cylindrical structure, constituted of combination of a plurality of cylindrically-shaped elements formed into a cylindrical shape by non-interruptedly connecting one end of a strand made of a biodegradable polymer to the other end of the strand, as disclosed in claim 9.

This cylindrically-shaped element is formed into a cylindrical shape by bending the strand repeatedly such that a linear part and a bend part alternate in sequence and non-interruptedly connecting one end of the strand to the other.

In one embodiment of the present invention, a plurality of cylindrically-shaped elements form a single cylindrical body by being held at their abutting parts with a holding member made of a polymer to be connected together.

As the luminal stent according to the present invention is formed by using a strand made of a biodegradable polymer, it is easy to make the stent carry one or more drugs. In the case of drug application of the stent, for example, a single cylindrical body may be made to carry multiple types of drug by application of several different drugs on a plurality of cylindrically-shaped elements constituting the cylindrical body.

### EFFECT OF THE INVENTION

The cylindrically-shaped element for a luminal stent according to the present invention is formed into a cylindrical shape by bending a single strand repeatedly such that a linear part and a bend part alternate in sequence, therefore it is constituted so as to be cylindrical structure with a predetermined height. By serially arranging and combining above-shaped cylindrically-shaped elements in the axial direction, a single cylindrical body having a length corresponding to the number of combined cylindrically-shaped elements can be obtained.

Therefore, by selecting the number of cylindrically-shaped elements to be combined, a luminal stent provided with a cylindrical body having a desired length corresponding to the number of combined cylindrically-shaped elements can be obtained. This facilitates formation of multiple types of luminal stent having different lengths appropriately.

Furthermore, since the cylindrically-shaped element is formed into a cylindrical shape by connecting ends of the strand which is repeatedly bent such that a linear part and a bend part alternate in sequence, a diameter of the cylindrical shape can be easily set by, for example, appropriately selecting a length of the strand to be used or changing the height of bends. Therefore, multiple types of cylindrical body having different diameters can be easily obtained.

As explained above, also diameters of the cylindrically-shaped elements of the present invention can be easily altered, production of luminal stents having freely-selected lengths and diameters is facilitated. Therefore a luminal stent suitable to a target vessel in a living body is easily produced.

Furthermore, since a plurality of the cylindrically-shaped elements according to the present invention constitute a single cylindrical body by partially connected at some of bends, the bends being bent in zigzag so that a linear part and a bend part alternate in sequence, displacement between adjacent cylindrically-shaped elements connected each other is easily obtained. This easy displacement between the cylindrically-shaped elements facilitates smooth deformation of the entire cylindrical body, which ensures flexibility of the luminal stent using this cylindrical body to deform easily in accordance with curvatures of vessels.

In addition, since the luminal stent applied the present invention forms a cylindrical body by combining a plurality of the cylindrically-shaped elements which constitute a part of the cylindrical body, the cylindrically-shaped elements being formed by bending a strand made of a biodegradable polymer repeatedly such that a linear part and a bend part alternate in sequence, the luminal stent can equally expand and scaffold the inner wall of a curved vessel.

Furthermore, since the luminal stent applied the present invention is entirely made of a biodegradable polymer, the safety for implantation in a living body is ensured. In particular, since the luminal stent of the present invention can be formed without use of adhesives including a solvent which might be harmful to a living body, no impurity other than biodegradable polymers is mixed in the stent ensuring the sufficient safety for implantation in a living body.

Furthermore, since the luminal stent according to the present invention is formed of a plurality of cylindrically-shaped elements, the luminal stent can easily carry multiple drugs in different types.

### BRIEF DESCRIPTION OF THE DRAWINGS

FIG. 1 is a side view showing a luminal stent formed by using cylindrically-shaped elements according to the present invention.
FIG. 2 is a perspective view showing a strand constituting a cylindrically-shaped element for a luminal stent according to the present invention.
FIG. 3 is a perspective view showing a cylindrically-shaped element constituting a cylindrical structure.
FIG. 4 is a perspective view showing a connecting portion of a cylindrically-shaped element, not part of the invention.
FIG. 5 is a perspective view showing another example of a connecting portion of a cylindrically-shaped element, not part of the invention.
FIG. 6 is a perspective view showing a connecting portion of a cylindrically-shaped element.
FIG. 7 is a plan view showing an example of bends a cylindrically-shaped element connected by holding members.

### DETAILED DESCRIPTION OF PREFERRED EMBODIMENTS

Referring to the drawings, embodiments of a cylindrically-shaped element and a luminal stent formed by using the cylindrically-shaped element are explained in detail.

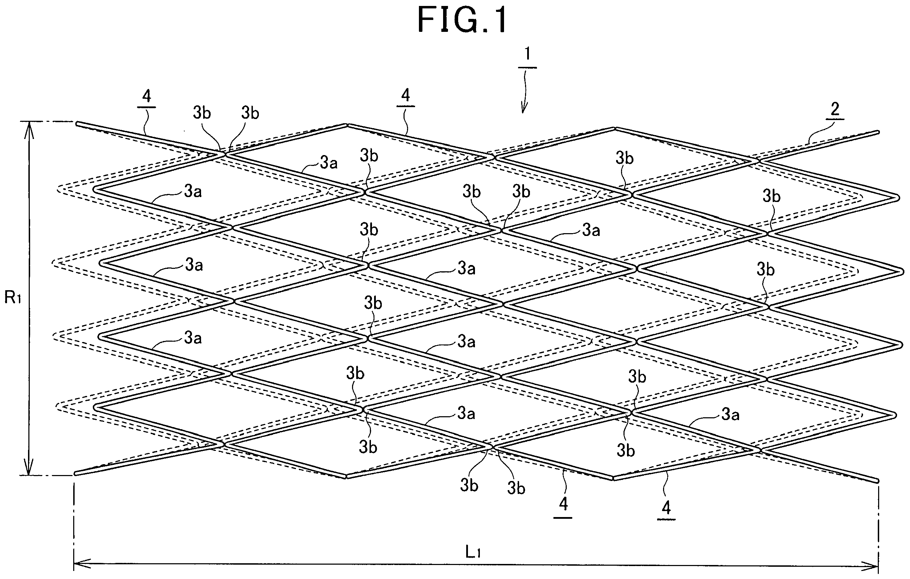

A cylindrically-shaped element according to the present invention is used to form a luminal stent to be implanted in a blood vessel such as coronary artery of a living body, and as shown in Fig.1 for example, this luminal stent 1 is constituted as a cylindrical body 2.

Dimensions of the luminal stent 1 according to the present invention are appropriately selected in accordance with a vessel of a living body in which the luminal stent 1 is to be implanted. For example, the luminal stent 1 intended for a blood vessel is configured to have an outer diameter R1 of 2 to 10 mm and a length L1 of 10 to 200 mm.

The cylindrical body 2 constituting the luminal stent 1 is formed by combining a plurality of cylindrically-shaped elements 4 according to the present invention, the cylindrically-shaped element 4 being formed of a strand 3 made of a biodegradable polymer. This strand 3 constituting the cylindrically-shaped element 4 is formed of a biodegradable polymer which, when implanted in a living body such as human body, does not cause adverse reaction therein. The biodegradable polymer used may be polylactic acid (PLLA), polyglycolic acid (PGA), polyglactin (copolymer of polyglycolic acid and polylactic acid), polydioxanone, polyglyconate (copolymer of trimethylene carbonate and glycolid), or a copolymer of polyglycolic acid or polylactic acid and ε -caprolactone. In addition, biodegradable polymers obtained by compounding two or more of these materials can be used.

The strand 3 is formed of a continuous monofilament composed of a biodegradable polymer and/or a multifilament composed of multiple monofilaments unified together. These monofilament and multifilament may be formed by, for example, melt-spinning a biodegradable polymer with a melt spinning apparatus.

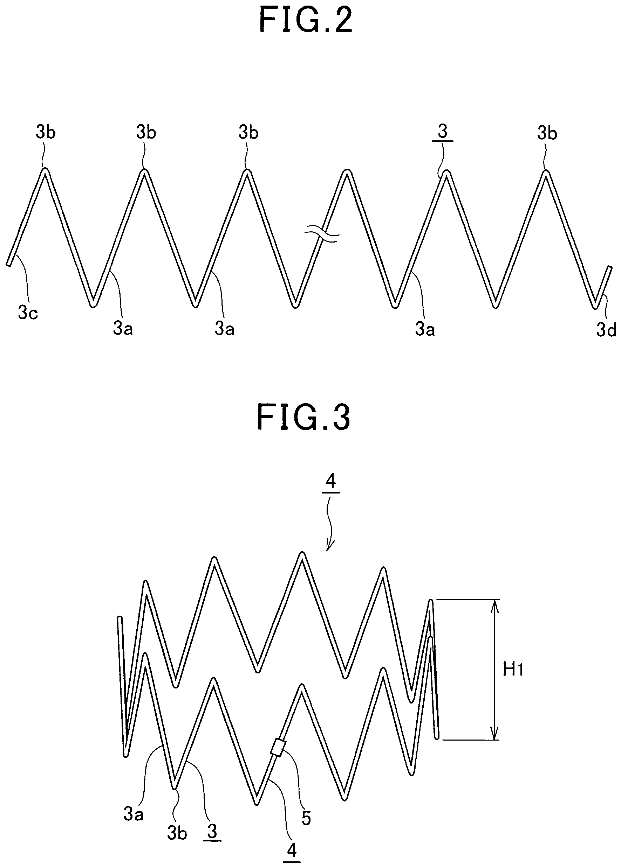

The cylindrically-shaped element 4 used herein is formed into a non-interrupted cylindrical shape by, as shown in Fig. 2, bending the single continuous strand 3 in a zigzag design such that a linear part 3a and a bend part 3b alternate in sequence, and connecting one end part 3c and the other end part 3d of this bent strand 3with a connecting member 5 as shown in Fig. 3.

The cylindrically-shaped element 4 according to the present invention is formed as a cylindrical shape having a height HI corresponding to a distance from one bend part 3b to the next bend part 3b, and as shown in Fig. 3, the region surrounded by the strand 3 has a cylindrical shape.

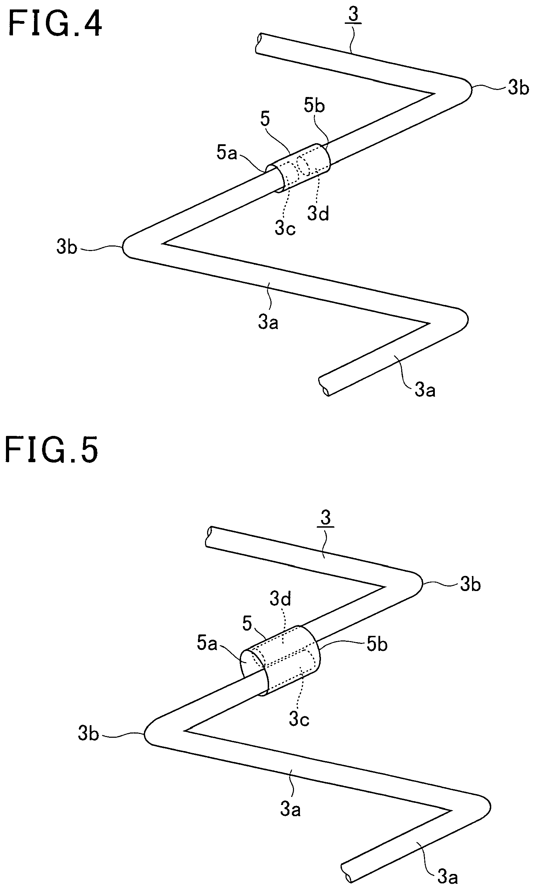

As an example, the single strand 3 bent in zigzag may be formed into a cylindrical shape by connecting one end part 3c and the other end part 3d with the connecting member 5 as shown in Fig. 4. This connecting member 5 is made of the same type of biodegradable polymer as the strand 3 and formed into a cylindrical shape.

The connecting member 5 for connecting one end part 3c and the other end part 3d of the strand 3 is cylindrical shape. By inserting one end part 3c of the strand 3 into one end part 5a of the connecting member 5, and the other end part 3d of the strand 3 into the other end part 5b of the connecting member 5, both ends 3c and 3d of the single strand 3 are non-interruptedly connected together. The connecting member 5, as shown in Fig. 4, are caulked to hold one end part 3c and the other end part 3d of the strand 3 by thermal shrinkage after heat application.

Thermal caulking of the connecting member 5 may be conducted by heating the connecting member 5 while pressurizing it. Pressurization enables the caulking at a lower temperature with a faster thermal shrinkage. Alternatively, during heating process the connecting member 5 may be heated so as to allow a part of it to melt. By being melted partially to weld the strand 3, the connecting member 5 non-interruptedly connects one end part 3c and the other end part 3d of the strand 3 more robustly.

However, in welding of the connecting member 5 to connect the strand 3, thermal deformation of the strand 3 is preferably avoided. That is, it is desirable to avoid changes in biodegradation characteristics of the strand 3due to influence of heat and pressure.

Now referring to Fig. 4, one end part 3c and the other end part 3d of the strand 3 are inserted into the connecting member 5 in abutting fashion without overlap to be non-interruptedly connected together with the connecting member 5. The strand 3 thus connected can form a cylindrical shape without conspicuous difference in thickness at the connected portion. That is, size of the connecting member 5 is sufficient unless hollow area of the connecting member 5 is large enough for accommodation of a single strand 3, therefore thickness change of the connected portion can be reduced.

It should be noted that, in order to reduce thickness at the connected portion, a section of one end part 3c and the other end part 3d of the strand 3 to be inserted in the connecting member 5 may be configured to be thinner than the other sections of the strand 3 such that this thinner area is to be held by the connecting member 5.

As another example, one end part 3c and the other end part 3d of the strand 3 to be connected with the connecting member 5 may be inserted into and connected by the connecting member 5 such that they overlap each other as shown in Fig. 5. In this way, more robust connection between one end part 3c and the other end part 3d of the strand 3 with the connecting member 5 can be ensured keeping a cylindrical shape more stable.

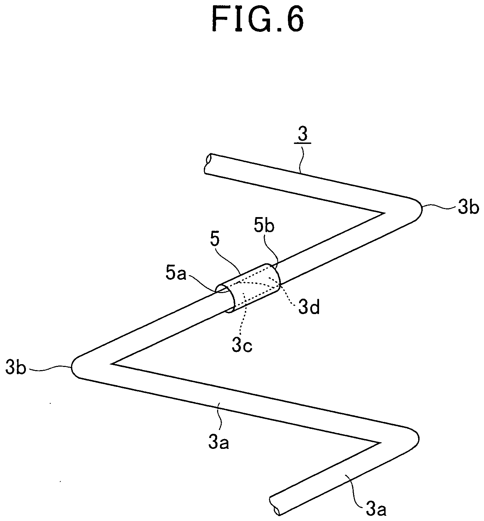

One end part 3c and the other end part 3d of the strand 3 is configured to have tapered ends as shown in Fig.6. By overlapping the tapered ends 3c and 3d while connecting them with the connecting member 5, thickness of the overlapped portion can be reduced avoiding thickness difference at the connected portion connected with the connecting member 5.

Referring to Fig.1, a plurality of cylindrically-shaped elements 4 formed into cylindrical shape as explained above, are arranged in multistage to form the single cylindrical body 2. In this process, the cylindrically-shaped element 4 and 4 axially adjacent to each other are arranged so that apexes of their bend part 3b and 3b are abutting.

A plurality of the cylindrically-shaped elements 4 arranged as shown in Fig.1 are connected together by thermally-welding an abutting pair of bend part 3b and 3b to form the single cylindrical body 2. This thermal welding of the bend parts 3b and 3b is achieved by melting a portion of the bend part 3b. Alternatively, a melted polymer may be provided between an abutting bend part 3b and 3b to achieve this thermal welding. A polymer used here for welding is preferably the same type of biodegradable polymer as the strand 3. Instead of above, abutting bend parts 3b and 3b may be bonded together with an adhesive.

It should be noted that there is no need to bond every abutting pair of bend parts 3b and 3b in the adjacent cylindrically-shaped elements 4 and 4; it might be enough to hold appropriate pairs of bend parts 3b and 3b. For example, every other pair may be bonded together.

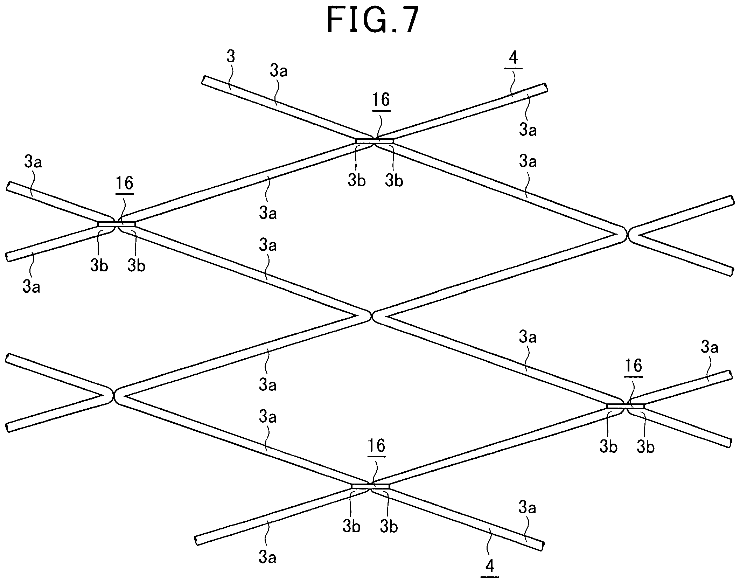

In a plurality of the cylindrically-shaped elements 4 and 4 arranged in multistage to form the single cylindrical body 2, abutting pairs of bend parts 3b and 3b can be joined with a method other than bonding; for example, the abutting pairs of bend parts 3b and 3b may be held by a holding members 16 as shown in Fig.7. The holding member 16 used herein may be formed into a ring shape or in a C-shape with an opened circumference. Again, there is no need to hold every abutting pair of bend parts 3b and 3b in the adjacent cylindrically-shaped elements 4 and 4 with the holding member 16; it might be enough to hold appropriate pairs of bend parts 3b and 3b. For example, as shown in Fig.7, every other pair may be held together with the holding member 16.

The holding member 16 used herein is formed of the same type of biodegradable polymer as the strand 3. The holding member 16 may be weld at a pinpoint so that this welded portion allows bend parts 3b and 3b of the strand 3 to melt in order to be connected together. In short, the holding member 16 is welded the strand 3while contacting with the strand 3 at a pinpoint. This welding is conducted by, for example, crimping the holding member 16 with a heating jig.

Although the embodiment explained above in which a plurality of cylindrically-shaped elements 4 and 4 arranged in multistage are connected by connecting their abutting bend parts 3b and 3b, linear parts 3a and 3a may be connected together by arranging them to overlap each other and holding them together with a holding member.

As explained above the cylindrically-shaped element 4 for a luminal stent according to the present invention is formed into cylindrical shape by bending a single strand 3 such that the linear part 3a and the bend part 3b alternate in sequence, therefore by serially arranging and combining an appropriate number of cylindrically-shaped elements 4 in the axial direction, configuration of the cylindrical body 2 having a length corresponding to the number of combined cylindrically-shaped elements 4 is achieved. Consequently, by selecting the number of cylindrically-shaped elements 4 to be combined, the luminal stent 1 comprising the cylindrical body 2 having an appropriate length corresponding to the number of combined cylindrically-shaped elements 4 can be easily obtained.

Furthermore, since the cylindrically-shaped element 4 is formed into a cylindrical shape by connecting the end parts 3c and 3d of the strand 3 wherein the linear part 3a and the bend part 3b alternate in sequence, a diameter of the cylindrical shape can be easily set by, for example, selecting a length of the strand 3 to be used or changing the number of bends appropriately. Therefore, multiple types of cylindrical body 2 having different diameters can be easily formed.

Furthermore, since a plurality of the cylindrically-shaped elements 4 according to the present invention constitute a single cylindrical body 2 by being connected together at some of their bends wherein the linear part 3 a and the bend part 3b are bent in zigzag to alternate in sequence, displacement between adjacent cylindrically-shaped elements 4 thus connected are smoothly obtained. This smooth displacement between the cylindrically-shaped elements 4 and 4 facilitates smooth deformation of the entire cylindrical body 2, which ensures flexibility of the luminal stent using this cylindrical body to deform easily in accordance with curvatures of vessels.

In addition, since the luminal stent 1 applied the present invention is formed as a cylindrical body 2 by combining a plurality of the cylindrically-shaped elements 4 constituting a part of the cylindrical body 2, the luminal stent 1 can equally expand and scaffold the inner wall of a curved vessel.

Furthermore, since the luminal stent 1 applied the present invention is entirely made of a biodegradable polymer; it disappears into a living body after implantation without need for removal procedure, which results in reduction of patient burden.

And more, since the luminal stent 1 according to the present invention is formed by using the strand 3 made of a biodegradable polymer, it can carry a drug easily. For example, a solvent of a drug may be applied onto the surface of the strand 3 constituting the cylindrically-shaped element 4. Alternatively a drug may be applied onto the surface of an assembled cylindrical body 2. Again in the latter case, a solvent of a drug may be used.

Furthermore, the single cylindrical body 2 may be made to carry multiple types of different drugs by application of different drugs on a plurality of cylindrically-shaped elements constituting the cylindrical body 2. In this case, the number of types of different drug can be increased up to the number of cylindrically-shaped elements 4 constituting cylindrical body 2.

As the embodiments described above are for illustrative purpose only, it is obvious that the number of cylindrically-shaped elements 4 connected and size of a cylindrically-shaped element 4 are altered in accordance with type and location of a vessel.

### INDUSTRIAL APPLICABILITY

The luminal stent formed of the cylindrically-shaped elements according to the present invention is implanted in a vessel of a living body, such as blood vessel including coronary artery, trachea or biliary duct to be used as a member for scaffolding the vessel from inside. The luminal stent formed of a biodegradable polymer material is implanted in a vessel in a living body and then disappears into a living body after a period of time.

## Claims

1. A cylindrically-shaped element (4) for a luminal stent (1) of cylindrical structure (2), comprising:
a sole strand (3) formed into cylindrical shape by bending said strand (3) repeatedly such that a linear part (3a) and a bend part (3b) alternate in sequence, and non-interruptedly connecting one end part (3c) of said strand (3) to the other end part (3d) of said strand (3),
**characterized in that** said strand (3) is made of a biodegradable polymer, and said end parts (3c, 3d) are tapered, wherein the tapered end parts (3c, 3d) being overlapped with each other to be connected together non-interruptedly.

2. The cylindrically-shaped element (4) for a luminal stent (1) according to claim 1, wherein both ends parts (3c, 3d) of said strand (3) are hold with a cylindrical connecting member (5) made of a biodegradable polymer in order to be connected together non-interruptedly.

3. The cylindrically-shaped element (4) for a luminal stent (1) according to claim 2, wherein said connecting member (5) is formed of the same type of biodegradable polymer used in said strand (3).

4. The cylindrically-shaped element (4) for a luminal stent (1) according to claim 2, wherein said connecting member (5) is formed of the same type of biodegradable polymer used in said strand (3) and caulked thermally to hold both end parts (3c, 3d) of said strand (3).

5. The cylindrically-shaped element (4) for a luminal stent (1) according to claim 2, wherein the connecting member (5) is formed of the same type of biodegradable polymer used in said strand (3) and welded at least partially to connect both end parts (3c, 3d) of said strand (3).

6. The cylindrically-shaped element (4) for a luminal stent (1) according to any of the preceding claims, wherein said strand (3) is a non-interrupted continuous monofilament.

7. The cylindrically-shaped element (4) for a luminal stent (1) according to any pf claims 1 to 5, wherein said strand (3) is a multifilament made of a plurality of monofilaments unified together.

8. The cylindrically-shaped element (4) for a luminal stent (1) according to any of the preceding claims, wherein said strand (3) is formed of one or more biodegradable polymer(s) selected from a group of polylactic acid (PLLA), polyglycolic acid (PGA), a copolymer of polyglycolic acid and polylactic acid, polydioxanone, a copolymer of trimethylene carbonate and glycolide, and a copolymer of polyglycolic acid or polylactic acid andε-caprolactone.

9. A luminal stent (1) having a cylindrical structure,:
wherein the cylindrical structure (2) is formed by a plurality of cylindrically-shaped elements (4) according to any of the preceding claims, wherein a plurality of said cylindrically-shaped elements (4) form a single cylindrical body (2) by being arranged in multistage in the axial direction and being connected at their abutting sections (3b).

10. The luminal stent (1) according to claim 9, wherein the connected abutting sections of a plurality of said cylindrically-shaped elements (4) are bend parts (3b) of a plurality of the cylindrically-shaped elements (4) arranged in multistage.

11. The luminal stent (1) according to claim 9, wherein the connected abutting sections of a plurality of said cylindrically-shaped elements (4) are linear parts (3a) of a plurality of the cylindrically-shaped elements (4) arranged in multistage.

12. The luminal stent (1) according to claim 9, wherein a plurality of said cylindrically-shaped elements (4) constituting said cylindrical body (2) are connected by holding their neighboring parts (3b) with a holding member (16) made of a polymer.

13. The luminal stent (1) according to claim 12, wherein said holding member (16) is formed of the same type of biodegradable polymer used in said strand (3).

14. The luminal stent (1) according to claim 9, wherein said cylindrically-shaped element (4) constituting said cylindrical body (2) carries a drug.

## Patentansprüche

1. Zylinderförmiges Element (4) für einen luminalen Stent (1) einer zylindrischen Struktur (2), umfassend:
einen einzigen Strang (3), der in eine zylindrische Form geformt wird, indem der Strang (3) wiederholt so gebogen wird, dass sich ein linearer Teil (3a) und ein Biegeteil (3b) nacheinander abwechseln, und ein Endteil (3c) des Strangs (3) ohne Unterbrechung mit dem anderen Endteil (3d) das Strangs (3) verbunden wird,
**dadurch gekennzeichnet, dass** der Strang (3) aus einem biologisch abbaubaren Polymer besteht und die Endteile (3c, 3d) verjüngt sind, wobei die verjüngten Endteile (3c, 3d) miteinander überlappt werden, um ohne Unterbrechung miteinander verbunden zu werden.

2. Zylinderförmiges Element (4) für einen luminalen Stent (1) nach Anspruch 1, wobei beide Endteile (3c, 3d) des Strangs (3) mit einem aus einem biologisch abbaubaren Polymer bestehenden zylindrischen Verbindungselement (5) gehalten werden, um ohne Unterbrechung miteinander verbunden zu werden.

3. Zylinderförmiges Element (4) für einen luminalen Stent (1) nach Anspruch 2, wobei das Verbindungselement (5) aus der gleichen, im Strang (3) verwendeten Art eines biologisch abbaubaren Polymers geschaffen ist.

4. Zylinderförmiges Element (4) für einen luminalen Stent (1) nach Anspruch 2, wobei das Verbindungselement (5) aus der gleichen, im Strang (3) verwendeten Art eines biologisch abbaubaren Polymers geschaffen ist und thermisch abgedichtet ist, um beide Endteile (3c, 3d) des Strangs (3) zu halten.

5. Zylinderförmiges Element (4) für einen luminalen Stent (1) nach Anspruch 2, wobei das Verbindungselement (5) aus der gleichen, im Strang (3) verwendeten Art eines biologisch abbaubaren Polymers geschaffen ist und zumindest teilweise verschweißt ist, um beide Endteile (3c, 3d) des Strangs (3) zu verbinden.

6. Zylinderförmiges Element (4) für einen luminalen Stent (1) nach einem der vorhergehenden Ansprüche, wobei der Strang (3) ein ununterbrochenes durchgehendes Monofilament ist.

7. Zylinderförmiges Element (4) für einen luminalen Stent (1) nach einem der Ansprüche 1 bis 5, wobei der Strang (3) ein Multifilament ist, das aus einer Vielzahl von miteinander vereinten Monofilamenten besteht.

8. Zylinderförmiges Element (4) für einen luminalen Stent (1) nach einem der vorhergehenden Ansprüche, wobei der Strang (3) aus einem oder mehr biologisch abbaubaren Polymer(en) geschaffen ist, die aus einer Gruppe aus Polymilchsäure (PLLA), Ployglycolidsäure (PGA), einem Coploymer aus Polyglycolidsäure und Polymilchsäure, Polydioxanon, einem Copolymer aus Trimethylencarbonat und Glycolid und einem Copolymer aus Polyglycolidsäure oder Polymilchsäure und ε-Caprolactons ausgewählt werden.

9. Luminaler Stent (1) mit einer zylindrischen Struktur,
wobei die zylindrische Struktur (2) von einer Vielzahl zylinderförmiger Elemente (4) nach einem der vorhergehenden Ansprüche gebildet wird, wobei eine Vielzahl der zylinderförmigen Elemente (4) einen einzigen zylindrischen Körper (2) bilden, indem sie in der axialen Richtung mehrstufig angeordnet sind und an ihren anstoßenden Sektionen (3b) verbunden sind.

10. Luminaler Stent (1) nach Anspruch 9, wobei die verbundenen anstoßenden Sektionen einer Vielzahl der zylinderförmigen Elemente (4) Biegeteile (3b) einer Vielzahl der zylinderförmigen Elemente (4) sind, die mehrstufig angeordnet sind.

11. Luminaler Stent (1) nach Anspruch 9, wobei die verbundenen anstoßenden Sektionen einer Vielzahl der zylinderförmigen Elemente (4) lineare Teile (3a) einer Vielzahl der zylinderförmigen Elemente (4) sind, die mehrstufig angeordnet sind.

12. Luminaler Stent (1) nach Anspruch 9, wobei eine Vielzahl der zylinderförmigen Elemente (4), die den zylindrischen Körper (2) bilden, verbunden sind, indem ihre benachbarten Teile (3b) mit einem aus einem biologisch abbaubaren Polymer bestehenden Halteglied (16) gehalten werden.

13. Luminaler Stent (1) nach Anspruch 12, wobei das Halteglied (16) auf der gleichen, im Strang (3) verwendeten Art eines biologisch abbaubaren Polymers geschaffen ist.

14. Luminaler Stent (1) nach Anspruch 9, wobei das den zylindrischen Körper (2) bildende zylinderförmige Element (4) ein Medikament trägt.

## Revendications

1. Élément de forme cylindrique (4) pour un stent vasculaire (1) à structure cylindrique (2), comprenant :
un seul fil (3) qui prend une forme cylindrique en étant plié (3) à plusieurs reprises de telle sorte qu'une partie linéaire (3a) et une partie formant coude (3b) alternent successivement, et qui relie sans interruption une partie d'extrémité (3c) du fil (3) à l'autre extrémité (3d) du fil (3),
**caractérisé en ce que** le fil (3) se compose d'un polymère biodégradable et les parties d'extrémité (3c, 3d) sont effilées, les parties d'extrémité effilées (3c, 3d) se recouvrant mutuellement afin d'être reliées sans interruption.

2. Élément de forme cylindrique (4) pour un stent vasculaire (1) selon la revendication 1, dans lequel les deux parties d'extrémité (3c, 3d) du fil (3) sont retenues avec un élément de liaison cylindrique (5) composé d'un polymère biodégradable afin d'être reliées sans interruption.

3. Élément de forme cylindrique (4) pour un stent vasculaire (1) selon la revendication 2, dans lequel l'élément de liaison (5) se compose du même type de polymère biodégradable que celui qui est utilisé dans le fil (3).

4. Élément de forme cylindrique (4) pour un stent vasculaire (1) selon la revendication 2, dans lequel l'élément de liaison (5) se compose du même type de polymère biodégradable que celui qui est utilisé dans le fil (3), et est maté thermiquement afin de retenir les deux parties d'extrémité (3c, 3d) du fil (3).

5. Élément de forme cylindrique (4) pour un stent vasculaire (1) selon la revendication 2, dans lequel l'élément de liaison (5) se compose du même type de polymère biodégradable que celui qui est utilisé dans le fil (3), et est soudé au moins partiellement afin de relier les deux parties d'extrémité (3c, 3d) du fil (3).

6. Élément de forme cylindrique (4) pour un stent vasculaire (1) selon l'une quelconque des revendications précédentes, dans lequel le fil (3) est un monofilament continu sans interruption.

7. Élément de forme cylindrique (4) pour un stent vasculaire (1) selon l'une quelconque des revendications 1 à 5, dans lequel le fil (3) est un multifilament composé de plusieurs monofilaments réunis.

8. Élément de forme cylindrique (4) pour un stent vasculaire (1) selon l'une quelconque des revendications précédentes, dans lequel le fil (3) se compose d'un ou plusieurs polymères biodégradables choisis dans un groupe constitué par un acide polylactique (PLLA), un acide polyglycolique (PGA), un copolymère d'acide polyglycolique et d'acide polylactique, un polydioxanone, un copolymère de carbonate de triméthylène et de glycolide, et un copolymère d'acide polyglycolique ou d'acide polylactique et de ε-caprolactone.

9. Stent vasculaire (1) comportant une structure cylindrique,
ladite structure cylindrique (2) étant formée par plusieurs éléments de forme cylindrique (4) selon l'une quelconque des revendications précédentes, plusieurs de ces éléments de forme cylindrique (4) formant un seul corps cylindrique (2) en étant disposés sur plusieurs étages dans le sens axial et en étant reliés, au niveau de leurs sections mises bout à bout (3b).

10. Stent vasculaire (1) selon la revendication 9, dans lequel les sections de plusieurs des éléments de forme cylindrique (4) qui sont mises à bout à bout et reliées sont des parties formant coudes (3b) de plusieurs des éléments de forme cylindrique (4) disposés sur plusieurs étages.

11. Stent vasculaire (1) selon la revendication 9, dans lequel les sections de plusieurs des éléments de forme cylindrique (4) qui sont mises à bout à bout et reliées sont des parties linéaires (3a) de plusieurs des éléments de forme cylindrique (4) disposés sur plusieurs étages.

12. Stent vasculaire (1) selon la revendication 9, dans lequel plusieurs éléments de forme cylindrique (4) constituant le corps cylindrique (2) sont reliés grâce à une fixation de leurs parties voisines (3b) avec un élément de retenue (16) composé d'un polymère.

13. Stent vasculaire (1) selon la revendication 12, dans lequel ledit élément de retenue (16) se compose du même type de polymère biodégradable que celui qui est utilisé dans le fil (3).

14. Stent vasculaire (1) selon la revendication 9, dans lequel l'élément de forme cylindrique (4) qui constitue le corps cylindrique (2) contient un médicament.
